Europäisches Patentamt

European Patent Office

Office européen des brevets

(11) Numéro de publication : **0 306 358 B1**

(19)

# (12) FASCICULE DE BREVET EUROPEEN

(45) Date de publication du fascicule du brevet :
**18.03.92 Bulletin 92/12**

(51) Int. Cl.$^5$ : **C07C 29/86,** C07C 29/80, C07C 31/04, C07C 31/08

(21) Numéro de dépôt : **88401869.8**

(22) Date de dépôt : **20.07.88**

(54) **Procédé de déshydratation d'un mélange aqueux d'alcools comprenant au moins du méthanol et de l'éthanol.**

(30) Priorité : **06.08.87 FR 8711253**

(43) Date de publication de la demande :
**08.03.89 Bulletin 89/10**

(45) Mention de la délivrance du brevet :
**18.03.92 Bulletin 92/12**

(84) Etats contractants désignés :
**BE DE GB IT NL**

(56) Documents cités :
**US-A- 2 614 971**
**US-A- 4 469 905**

(73) Titulaire : **INSTITUT FRANCAIS DU PETROLE**
**4, Avenue de Bois Préau**
**F-92502 Rueil-Malmaison (FR)**

(72) Inventeur : **Pucci, Annick**
**89, rue Eugène Labiche**
**F-78290 Croissy sur Seine (FR)**
Inventeur : **Mikitenko, Paul**
**25, Orée de Marly**
**F-78590 Noisy le Roi (FR)**

EP 0 306 358 B1

## Description

L'invention concerne un procédé utilisable en continu, de déshydratation d'un mélange de méthanol et d'éthanol pouvant renfermer, en outre, des alcools plus lourds. Le procédé s'applique en particulier à un mélange d'alcools $C_1$-$C_6$ contenant du méthanol et en proportions décroissantes, des alcools plus lourds, des traces d'hydrocarbures et des produits oxygénés divers, obtenus par synthèse à partir du gaz $CO+H_2$. Ce gaz peut être produit à partir de nombreuses sources telles que le charbon, les résidus lourds du pétrole, la biomasse et plus récemment par conversion du gaz naturel. Dans la perspective de l'utilisation de carburants sans plomb dans les moteurs, ces mélanges d'alcools peuvent être utilisés pour augmenter l'indice d'octane des essences de base, la présence d'alcools lourds évitant par ailleurs la démixtion en deux phases liquides en case d'introduction accidentelle d'eau.

Le mécanisme de la réaction catalytique de production de ces alcools obéit à une distribution de SCHULTZ-FLORY de sorte que la répartition pondérale des principaux constituants de ces mélanges est d'environ 50 à 70 % pour le méthanol, 15 à 20 % pour l'éthanol, 7 à 10 % pour le propanol et l'isopropanol, 4 à 5 % pour le butanol et ses isomères et 2 à 4 % pour les alcools $C_5^+$ contenant au moins 5 atomes de carbone auxquels s'ajoutent l'eau associée, des traces d'hydrocarbures et des produits oxygénés divers. On peut par exemple obtenir des mélanges contenant de 30 à 45 % poids d'alcools $C_2^+$ et moins de 1 % poids d'alcools $C_8^+$. Par contre la teneur en eau peut varier dans de larges proportions (2,5 à 30 %) selon la quantité de gaz carbonique admise dans le gaz de synthèse à l'entrée du réacteur.

Le présent procédé s'applique tout particulièrement aux mélanges renfermant 20 à 90 % en poids de méthanol, 5 à 50 % en poids d'éthanol et 1 à 40 % en poids d'eau. Il peut également renfermer des alcools supérieurs.

L'objet de la présente invention est de proposer une méthode de déshydratation du mélange d'alcools, applicable industriellement à grande échelle pour fournir un produit d'une teneur en eau inférieure à 0,5 % poids, susceptible d'être additionné aux essences sans plomb sans risque de démixtion.

Aussi un des buts de la présente invention est de mettre en oeuvre une technique économe en matériel et en énergie et adaptable à la teneur en eau du mélange d'alcools à traiter en ce sens, que pour des concentrations en eau élevées, le procédé associe extraction et distillation et que, pour des concentrations plus faibles, l'étape d'extraction peut être supprimée.

Un but supplémentaire est d'offrir un procédé utilisant le même agent comme agent d'extraction et comme agent déshydratant. L'invention est basée sur l'emploi d'un hydrocarbure ayant de 2 à 6 atomes de carbone tel que l'éthane, le propane, l'isobutane, le butane, un pentane ou un hexane ou les oléfines correspondantes éthylène, propylène, butènes, pentènes, hexènes individuellement ou en mélange et préférentiellement une coupe $C_3$ ou $C_4$ et avantageusement, le propane, le butane ou l'isobutane tels qu'on peut les trouver sur le site même de la raffinerie. L'utilisation de ces hydrocarbures permet d'associer d'une part l'extraction avantageuse d'alcools en solution aqueuse et d'autre part leur purification finale par distillation déshydratante en raison d'un hétéroazéotrope hydrocarbure eau.

Enfin de façon surprenante et inattendue et sans atteindre des niveaux de température ou de pression trop élevés, le procédé conduit à une récupération avantageuse de l'énergie. Une caractéristique de l'invention réside en ce que la chaleur autre que celle nécessaire au rebouillage de la première colonne de déméthanolisation du mélange d'alcools, est en grande partie fournie par la condensation des vapeurs de tête de ladite colonne : cette chaleur est utilisée en particulier dans la colonne de récupération de l'hydrocarbure.

Le procédé de l'invention comprend les étapes essentielles suivantes :

a) on fractionne le mélange aqueux d'alcools, dans une première zone de fractionnement, par distillation sous une pression $P_1$ au moins égale à la pression atmosphérique ; on recueille en tête les vapeurs d'au moins 95 % du méthanol et de 0 à 65 % de l'éthanol dudit mélange aqueux d'alcools et on condense lesdites vapeurs en transmettant au moins une partie de la chaleur de condensation à la seconde zone de fractionnement, comme indiqué plus loin ; on soutire en fond un courant liquide d'alcools et d'eau appauvri (ou débarrassé) de méthanol ;

b) on ajoute au moins un hydrocarbure ayant de 2 à 6 atomes de carbone audit courant liquide d'alcools et d'eau et on fractionne le mélange résultant, renfermant ledit hydrocarbure, par distillation dans une seconde zone de fractionnement sous une pression $P_2$ telle que ladite chaleur de condensation permette la distillation d'au moins la majeure partie de l'hydrocarbure dans la seconde zone de fractionnement, et on recueille en tête un distillat comprenant essentiellement au moins la majeure partie dudit hydrocarbure et de l'eau et, en fond, un courant liquide renfermant les alcools substantiellement déshydratés, et on sépare l'eau de l'hydrocarbure,et

c) on mélange au moins une partie du condensat de la première zone de fractionnement avec au moins une partie du courant liquide soutiré en fond de la seconde zone de fractionnement.

De préférence on règle la première zone de fractionnement de manière à recueillir en tête au moins 98 % du méthanol et 5 à 55 % de l'éthanol. On est cependant limité par la spécification en eau à respecter pour le distillat qui est généralement inférieure à 1 % en poids, ce qui peut obliger à limiter la proportion d'éthanol distillé puisque l'éthanol entraîne inévitablement de l'eau par azéotropie, contrairement au méthanol.

De préférence de 1 à 15 %, plus particulièrement de 2 à 8 %, de l'hydrocarbure introduit dans la seconde zone de fractionnement est déchargé en mélange avec le courant liquide de fond de ladite seconde zone de fractionnement et soumis à une distillation et/ou strippage complémentaire, de préférence sous pression moins élevée que celle de la seconde zone de fractionnement, de manière à éliminer substantiellement l'hydrocarbure résiduel. On évite ainsi d'avoir une température trop élevée au rebouilleur de la seconde zone de fractionnement, qui rendrait impossible le chauffage par récupération de la chaleur de condensation des vapeurs de la première zone de fractionnement. Dans ce cas, c'est le produit de fond de cette distillation complémentaire qui est mélangé au condensat de la première zone de fractionnement.

Le procédé, tel que décrit ci-dessus, convient particulièrement bien pour des teneurs en eau du mélange de départ de 1 à 5 % en poids.

Selon un mode de mise en oeuvre particulièrement adapté aux teneurs élevées d'eau dans le mélange d'alcools de départ (par exemple supérieures à 3,5 % en poids), on soumet à la distillation (étape a) le mélange d'alcools à fractionner pour éliminer avantageusement le méthanol anhydre des autres alcools qui présentent un azéotrope avec l'eau. Sauf dans le cas où l'on désire récupérer le méthanol séparément, il est préféré pour l'économie du procédé de récupérer dans le distillat en plus du méthanol une partie significative - jusqu'à 65 % environ - de l'éthanol contenu dans la charge sans dépasser toutefois la concentration d'eau tolérée pour un usage carburant. La colonne opère à la pression atmosphérique ou sous une pression légèrement plus élevée, de préférence inférieure à 5 bars. Avantageusement, et sans utiliser une technique de compression mécanique des vapeurs, le niveau de température est tel qu'en se condensant ces vapeurs de tête fournissent une partie importante de la chaleur nécessaire au rebouillage de la seconde zone de fractionnement. On produit alors en distillat un mélange méthanol, éthanol déshydraté qui sera ultérieurement mélangé avec le reste de l'éthanol et avec les alcools plus lourds déshydratés, et en fond de tour une solution d'alcools supérieurs $C_2^+$ contenant le plus souvent de 2 à 50 % poids d'eau, par exemple de 3 à 25 % poids d'eau.

On introduit ladite solution déméthanolée dans un extracteur de préférence du type à contre courant, où elle est contactée par un des solvants précités. Les équilibres mis en jeu peuvent être de nature liquide-liquide ou liquide-fluide si le solvant est porté dans des conditions de pression et de température supérieures à celles de son point critique. Avantageusement l'extracteur fonctionne entre 60 et 150 °C et par exemple à la température du fluide d'alimentation. La pression doit être suffisante pour maintenir la phase hydrocarbure à l'état liquide. Des pressions de 1 à 50 bars sont donc utilisables. Avantageusement, le taux de solvant ne dépasse pas 3. Lorsque le taux d'extraction des alcools est voisin de 100 % le raffinat aqueux est directement évacué. Si le rendement de l'extraction est seulement de 90 à 99 % , le raffinat peut être envoyé dans une petite colonne de distillation auxiliaire qui produit comme distillat des alcools à faible teneur en eau qui peuvent être recyclés à l'extraction tandis qu'en fond l'eau résiduelle est éliminée. Cette colonne auxiliaire peut fonctionner par exemple sous la pression atmosphérique.

On envoie la phase d'extrait contenant en mélange la solution d'alcools $C_2^+$ partiellement déshydratés et le solvant d'extraction à l'étape de régénération. Il est bien connu que dans un procédé d'extraction, l'étape de régénération du solvant est souvent très coûteuse. Selon la présente invention, au moins la majeure partie de la chaleur nécessaire à cette étape est fournie par la condensation des vapeurs produites en tête à l'étape (a). Cela est rendu possible parce que la pression de la seconde zone de fractionnement est choisie de sorte que la température du rebouilleur soit inférieure, par exemple d'au moins 5 °C, à la température de condensation des vapeurs de la première zone de fractionnement. Dans ce but, ladite colonne de régénération est de préférence réglée de sorte qu'on laisse fuir dans le mélange d'alcools récupéré en fond la quantité de solvant nécessaire à l'ajustement de la température au rebouilleur et pour que la condensation du distillat en tête se fasse à 20 °C ou au-dessus et ne nécessite donc pas l'utilisation d'un cycle frigorifique. Les pressions, selon l'hydrocarbure, peuvent se situer de 1 à 50 bars, par exemple 5-30 bars avec le propane et 3-20 bars avec le butane. Par ailleurs l'eau résiduelle entraînée par hétéroazéotropie avec le solvant se sépare au ballon de reflux de ladite colonne. Dans le cas de l'utilisation d'un hydrocarbure tel que l'éthane ou le propane, la phase aqueuse soutirée au décanteur,exempte d'alcools, peut être directement éliminée. Avec l'emploi d'un solvant plus lourd que le propane la quantité d'alcools contenue dans la phase aqueuse peut nécessiter un fractionnement complémentaire. La phase organique, très majoritairement le solvant, est pour partie retournée à l'étape d'extraction, et pour partie renvoyée à la colonne en reflux.

Enfin l'adjonction d'une colonne annexe d'épuration est préférée pour débarrasser le mélange d'alcools $C_2^+$ déshydraté du solvant. La pression opératoire est égale ou de préférence inférieure à la pression de la colonne précédemment décrite. On opérera avantageusement à la pression atmosphérique. Les vapeurs produites en distillat peuvent être recomprimées et retournées à la seconde zone de fractionnement. La chaleur à fournir à cette colonne est de 3 à 10 fois moins importante que celle nécessaire au rebouillage de la précédente colonne (seconde étape de fractionnement).

Les alcools $C_2^+$ ainsi produits, additionnés au condensat fourni par la distillation décrite à l'étape (a) réalisent un mélange d'alcools pouvant contenir moins de 0,5 % en poids d'eau. Par exemple à partir d'un mélange d'alcools $C_1$-$C_5^+$ tel que défini plus haut, on peut obtenir un mélange de 50 à 70 % de méthanol, 15 à 20 % d'éthanol, 7 à 10 % de propanol, 4 à 5 % de butanol et 3 à 4 % d'alcools $C_5^+$ et moins de 0,5 % d'eau. Ce mélange convient pour un usage carburant.

La figure 1 illustre un premier mode de réalisation comportant une extraction par solvant et une séparation d'eau.

La figure 2 illustre un mode de réalisation simplifié, ne comportant pas d'extraction par solvant.

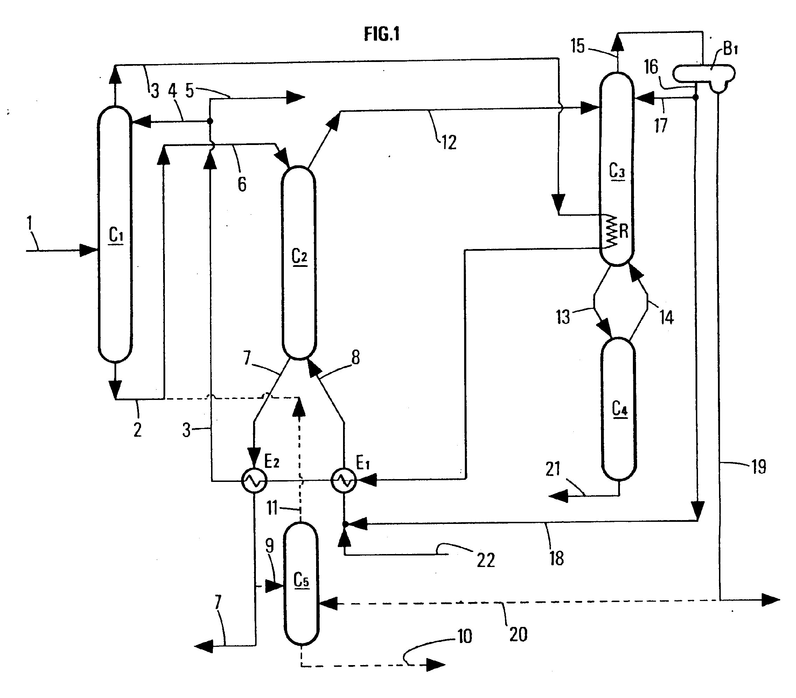

De façon surprenante et inattendue, un tel schéma de procédé ne faisant intervenir que la distillation est préféré pour l'économie d'énergie et de matériel qu'il entraîne dès que la teneur en eau du mélange d'alcool descend en dessous d'environ 3 à 5 % en poids (par exemple 1-3,5 % en poids). En comparaison avec le schéma de procédé de la figure 1, les colonnes de distillation sont conservées tandis que l'extracteur est supprimé. Comme dans le premier mode de réalisation de l'invention, les alcools $C_2^+$ déshydratés produits en fond contiennent la quantité de solvant nécessaire à l'ajustement de la température du rebouilleur tandis que la phase aqueuse qui démixe est évacuée au décanteur de tête. Les exemples que suivent illustrent l'invention :

## EXEMPLE 1

On opère suivant le schéma de la figure 1 pour déshydrater au propane un mélange d'alccols dont la composition pondérale est donnée dans le tableau I. On introduit au milieu de la colonne C1 de 50 plateaux au total, par la ligne 1, le mélange à déshydrater à raison de 327 kg/h. Ladite colonne opére sous une pression moyenne de 1,2 bar et avec un taux de reflux de 1,5. On recueille en tête 620 kg/h de vapeur à 75 °C (ligne 3) contenant la quasi totalité du méthanol de l'alimentation et la moitié de l'éthanol avec une concentration en eau de 0,2 % poids. En se condensant (échangeur R) entre 75 et 70 °C, elles permettent de rebouillir la colonne C3 de régénération du propane et de réchauffer le solvant recyclé (échangeur E1) et la phase raffinat (échangeur E2) circulant respectivement dans les lignes 8 et 7. Le distillat liquide est alors évacué par la conduite 5 et stocké. Un partie est renvoyée comme reflux (4). Simultanément on récupère en fond de colonne de déméthanolisation (ligne 2) un mélange d'alcools $C_2^+$ à 20 % poids d'eau, qui alimente (ligne 6) la colonne d'extraction C2 à raison de 100 kg/h. Cette colonne fonctionne à 80 °C sous 35 bars. Elle reçoit en fond par la conduite 8 un débit de propane d'environ 285 kg/h. Le taux d'extraction de l'ensemble des alcools s'élève à 95 % de sorte qu'il est nécessaire d'envoyer par la ligne 9 sur la colonne C5 le raffinat pour récupérer son contenu d'alcools à un niveau de concentration de 80 % poids et les recycler par la ligne 11 à l'extraction. La colonne C5 évacue par ailleurs par la ligne 10, 14 kg/h d'eau à la pression atmosphérique sous 100 °C. Un petit appoint d'énergie de 10 mégajoules/h est fourni au rebouilleur. La phase extrait à 1,3 % poids d'eau, 78 % de propane et qui contient 95 % des alcools admis à l'entrée de l'extracteur est envoyée par la conduite 12 à la colonne C3 de régénération du solvant avec un débit de 367 kg/h. La colonne C3 reçoit aussi au rebouilleur R une vapeur comprimée et surchauffée issue de C4 (ligne 14).

La colonne C3 fonctionne sous une pression moyenne de 10 bars et à 68 °C au rebouilleur R. Elle produit :
– d'une part en fond de tour par la ligne 13 le mélange d'alcools $C_2^+$ déshydraté contenant encore 16 % poids de propane sous une température de 68 °C (soit environ 5 % de l'hydrocarbure introduit),
– d'autre part en tête des vapeurs (ligne 15) qui décantent à 25 °C au ballon de reflux B1 de sorte que 4,8 kg/h d'eau soient éliminés par la ligne 19 et le propane renvoyé partiellement en reflux par les lignes 16 et 17 à raison de 145 kg/h et majoritairement à l'extracteur C2 par les conduites 18 et 8. La ligne 20 n'est pas utilisée (elle est employée lorsque le solvant est plus lourd que le propane).

La dernière colonne C4 reçoit le fond de tour de C3 par la ligne 13 et lui renvoie les vapeurs de propane concentré (ligne 14) produites par la section d'épuisement de cette dernière. Elle nécessite un apport d'énergie thermique de 25 mégajoules/ h. Fonctionnant sous 1 bar elle produit 76 kg/h de mélange d'alcools $C_2^+$ déshydraté (ligne 21) à 99 °C avec moins de 500 ppm poids de propane et moins de 2000 ppm poids d'eau. Ce mélange combiné avec le distillat (5) issu de la déméthanolisation fournit 308 kg/h de produit déshydraté, contenant les mêmes alcools en mêmes proportions que ceux de l'alimentation. Ces alcools conviennent aux exigences d'un usage carburant.

Un appoint mineur de propane peut être effectué par la conduite 22 pour compenser les pertes en solvant.

La rendement global du procédé en alcools est quasiment de 100 % et la consommation énergétique qui se limite pour l'essentiel à la chaleur à fournir pour les rebouillages de la colonne C1 majoritairement et pour une plus faible part des colonnes C4 et C5 se monte à 0,05 tonne d'équivalent pétrole (TEP)/tonne de produit déshydraté. En particulier la chaleur délivrée dans l'échangeur R par la condensation des vapeurs de la colonne C1 est plus que suffisante pour assurer le fonctionnement de la colonne C3.

## EXEMPLE 2

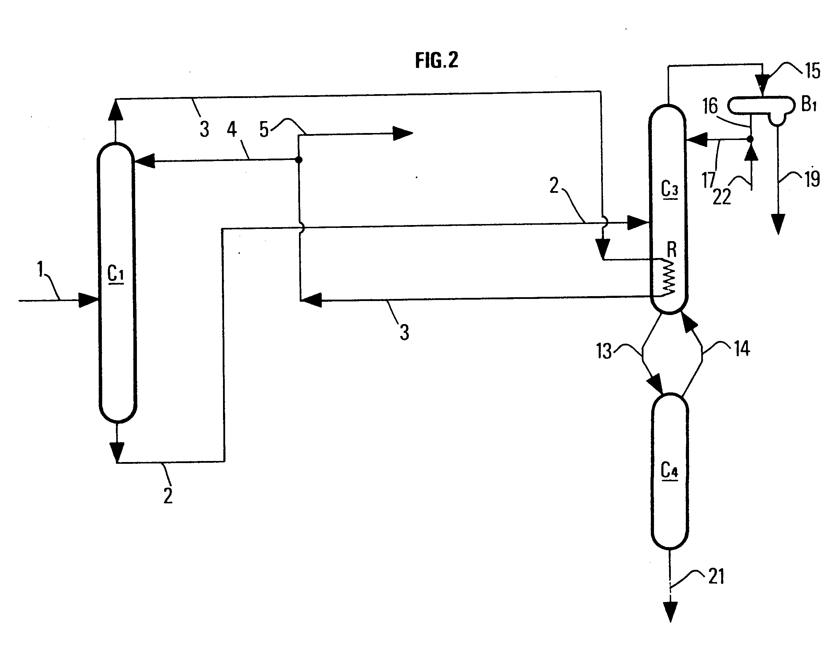

On opère la déshydratation d'un mélange d'alcools de synthèse dont la teneur en eau est de 3,3 % poids suivant le schéma de procédé présenté sur la figure 2. La composition détaillée de la charge figure dans le tableau I. Le propane est encore choisi comme agent déshydratant. Les colonnes à distiller, la position des alimentations, les niveaux de pression et de température sont en tout point identiques à ceux décrits dans l'exemple 1. Les seules modifications liées à la suppression de l'extracteur concernent l'alimentation de la colonne de régénération C3 qui reçoit directement le fond de tour de déméthanolisation (C1) et la récupération de chaleur qui porte exclusivement sur la rebouillage de ladite colonne.

Ainsi à partir de 338,8 kg/h de mélange d'alcools envoyé sur C1 (ligne 1) on produit un distillat vapeur (ligne 3) qui permet le rebouillage de C3 et fournit après condensation 248 kg/h d'un mélange contenant le méthanol et la moitié de l'éthanol de la charge à une concentration de 0,2 % poids d'eau moyennant un taux de reflux identique. On récupère en fond de colonne de déméthanolisation (ligne 2) le mélange d'alcools $C_2^+$ à 11,9 % poids d'eau qui alimente directement la colonne azéotropique C3 avec un débit de 90,8 kg/h. Un volant de propane de 1000 kg/h permet d'évacuer au ballon décanteur B1 10,8 kg/h de phase aqueuse pratiquement exempte d'alcools par la conduite 19. Le mélange d'alcools $C_2^+$ à 20 % mole de propane est récupéré en fond de colonne. La colonne C3 fonctionne en fond à 10 bars et 68 °C. Identiquement à ce qui est décrit dans l'exemple 1, le solvant résiduel est chassé dans la colonne atmosphérique C4 qui produit par la conduite 21, 80 kg/h d'alcools $C_2^+$ déshydratés.

Le mélange global est reconstitué à partir des produits évacués par les lignes 5 et 21.

Pour une même pureté d'alcools produits que dans l'exemple 1, la consommation énergétique est inférieure à 0,05 TEP/tonne, l'économie en équipement est significative et le rendement de 100 % conservé. La totalité de la chaleur nécessaire à la colonne C3 est fournie par la condensation des vapeurs de la colonne C1.

## Tableau I

### Composition pondérale du mélange d'alcools à l'alimentation

|  | Exemple I | Exemple II |
|---|---|---|
| Eau | 5,9 % | 3,3 % |
| Methanol | 62,9 % | 64,7 % |
| Ethanol | 16,2 % | 16,8 % |
| Propanol | 7,9 % | 8,0 % |
| Butanol | 4,5 % | 4,5 % |
| Pentanol | 1,7 % | 1,8 % |
| Hexanol et autres alcools + lourds | 0,9 % | 0,9 % |

**Revendications**

1. Procédé de déshydratation d'un mélange aqueux d'alcools renfermant au moins du méthanol et de l'éthanol, caractérisé en ce qu'il comporte les étapes suivantes :

a) on fractionne le mélange aqueux d'alcools, dans une première zone de fractionnement, par distillation sous une pression $P_1$ au moins égale à la pression atmosphérique ; on recueille en tête les vapeurs d'au moins 95 % du méthanol et de 0 à 65 % de l'éthanol dudit mélange aqueux d'alcools et on condense lesdites vapeurs en transmettant au moins une partie de la chaleur de condensation à la seconde zone de fractionnement, comme indiqué plus loin ; on soutire en fond un courant liquide d'alcools et d'eau appauvri (ou débarrassé) de méthanol ;

b) on ajoute au moins un hydrocarbure ayant de 2 à 6 atomes de carbone audit courant liquide d'alcools et d'eau et on fractionne le mélange résultant, renfermant ledit hydrocarbure, par distillation dans une seconde zone de fractionnement sous une pression $P_2$ telle que ladite chaleur de condensation permette la distillation d'au moins la majeure partie de l'hydrocarbure dans la seconde zone de fractionnement, et on receuille en tête un distillat comprenant essentiellement au moins la majeure partie dudit hydrocarbure et de l'eau et, en fond, un courant liquide renfermant les alcools substantiellement déshydratés, et on sépare l'eau de l'hydrocarbure, et

c) on mélange au moins une partie du condensat de la première zone de fractionnement avec au moins une partie du courant liquide soutiré en fond de la seconde zone de fractionnement.

2. Procédé selon la revendication 1 dans lequel, à l'étape (b), avant d'envoyer le mélange d'hydrocarbure et du courant d'alcools et d'eau à la seconde zone de fractionnement, on décante une phase aqueuse qui s'est formée lors de l'ajout d'hydrocarbure, on élimine ladite phase aqueuse, et on envoie à la seconde zone de fractionnement le mélange débarassé de ladite phase aqueuse.

3. Procédé selon la revendication 2, dans lequel l'ajout d'hydrocarbure s'effectue par mise en contact à contre-courant dudit hydrocarbure avec ledit courant liquide d'alcools et d'eau.

4. Procédé selon l'une des revendications 1 à 3, dans lequel on règle la première zone de fractionnement pour recueillir en tête au moins 98 % du méthanol et 10 à 55 % de l'éthanol.

5. Procédé selon l'une des revendication 1 à 4 dans lequel l'hydrocarbure est essentiellement du propane et où la pression est de 1-5 bars dans la première zone de fractionnement et 5-30 bars dans la seconde zone de fractionnement.

6. Procédé selon l'une des revendications 1 à 4 dans lequel l'hydrocarbure est essentiellement du butane et où la pression est de 1-5 bars dans la première zone de fractionnement et 3-20 bars dans la seconde zone de fractionnement.

7. Procédé selon l'une des revendications 1 à 6, dans lequel le mélange aqueux d'alcools renferme de 1 à 40 % en poids d'eau.

8. Procédé selon l'une des revendications 1 à 6, dans lequel le mélange aqueux d'alcools renferme de 1 à 40 % en poids d'eau et de 99 à 60 % en poids d'alcools, les alcools comprennant en poids, pour 100 parties, 50-70 % de méthanol, 15-20 % d'éthanol, 7-10 % de propanol, 4-5 % de butanol et 2.4 % d'alcools ayant au moins 5 atomes de carbone.

9. Procédé selon l'une des revendications 1 à 8, dans lequel on règle la distillation de la seconde zone de fractionnement de manière à distiller 85-99 % de l'hydrocarbure chargé et à laisser 1 à 15 % de l'hydrocarbure chargé dans le courant liquide de fond, renfermant les alcools substantiellement déshydratés, on soumet à une distillation complémentaire ledit courant liquide sous une pression moins élevée que celle de la seconde zone de fractionnement, on recueille en tête essentiellement l'hydrocarbure résiduel et en fond les alcools anhydres essentiellement débarrassés de l'hydrocarbure.

10. Utilisation du mélange obtenu à l'étape (c) de l'une quelconque des revendications 1 à 9 comme carburant ou constituant de carburant pour moteurs.

**Patentansprüche**

1. Verfahren zur Dehydratation einer wäßrigen Mischung von Alkoholen, die mindestens Methanol und Ethanol enthält, dadurch gekennzeichnet, daß sie die folgenden Stufen umfaßt:

a) man fraktioniert die wäßrige Mischung von Alkoholen in einer ersten Fraktionierungszone durch Destillation unter einem Druck $P_1$, der mindestens gleich dem Atmosphärendruck ist; man sammelt am Kopf die Dämpfe von mindestens 95 % des Methanols und 0 bis 65 % des Ethanols der wäßrigen Alkoholmischung

EP 0 306 358 B1

und kondensiert diese Dämpfe, indem man mindestens einen Teil der Kondensationswärme in die zweite Fraktionierungszone wie weiter unten angegeben überführt; man zieht am Boden einen Flüssigkeitsstrom von Alkoholen und Wasser, der an Methanol verarmt (oder davon befreit) ist, ab;

b) man gibt mindestens einen Kohlenwasserstoff mit 2 bis 6 Kohlenstoffatomen dem Flüssigkeitsstrom von Alkoholen und Wasser zu und fraktioniert die resultierende Mischung, die den Kohlenwasserstoff enthält, durch Destillation in einer zweiten Fraktionierungszone unter einem Druck $P_2$ in der Weise, daß die Kondensationswärme die Destillation mindestens des Hauptteils des Kohlenwasserstoffs in der zweiten Fraktionierungszone erlaubt, und man sammelt am Kopf ein Destillat, das im wesentlichen mindestens den Hauptteil des Kohlenwasserstoffs und des Wassers enthält, und am Boden einen Flüssigkeitsstrom, der die im wesentlichen dehydratisierten Alkohole enthält, und man trennt das Wasser von dem Kohlenwasserstoff, und

c) man mischt mindestens einen Teil des Kondensats der ersten Fraktionierungszone mit mindestens einem Teil des am Boden der zweiten Fraktionierungszone abgezogenen Flüssigkeitsstroms.

2. Verfahren nach Anspruch 1, bei dem man in der Stufe (b) vor dem Einführen der Mischung aus dem Kohlenwasserstoff und dem Strom von Alkoholen und Wasser in die zweite Fraktionierungszone eine wäßrige Phase dekantiert, die sich bei der Zugabe des Kohlenwasserstoffs gebildet hat, diese wäßrige Phase eliminiert und die von dieser wäßrigen Phase befreite Mischung in die zweite Fraktionierungszone überführt.

3. Verfahren nach Anspruch 2, bei dem die Zugabe des Kohlenwasserstoffs in der Weise erfolgt, daß man den Kohlenwasserstoff mit dem Flüssigkeitsstrom von Alkoholen und Wasser im Gegenstrom in Kontakt bringt.

4. Verfahren nach einem der Ansprüche 1 bis 3, bei dem man die erste Fraktionierungszone so einstellt, daß am Kopf mindestens 98 % des Methanols und 10 bis 55 % des Ethanols gesammelt werden.

5. Verfahren nach einem der Ansprüche 1 bis 4, bei dem der Kohlenwasserstoff im wesentlichen Propan ist und bei dem der Druck in der ersten Fraktionierungszone 1 bis 5 bar und in der zweiten Fraktionierungszone 5 bis 30 bar beträgt.

6. Verfahren nach einem der Ansprüche 1 bis 4, bei dem der Kohlenwasserstoff im wesentlichen Butan ist und bei dem der Druck in der ersten Fraktionierungszone 1 bis 5 bar und in der zweiten Fraktionierungszone 3 bis 20 bar beträgt.

7. Verfahren nach einem der Ansprüche 1 bis 6, bei dem die wäßrige Alkoholmischung 1 bis 40 Gew.-% Wasser enthält.

8. Verfahren nach einem der Ansprüche 1 bis 6, bei dem die wäßrige Alkoholmischung 1 bis 40 Gew.-% Wasser und 99 bis 60 Gew.-% Alkohole enthält, wobei die Alkohole, bezogen auf 100 Teile, umfassen 50 bis 70 Gew.-% Methanol, 15 bis 20 Gew.-% Ethanol, 7 bis 10 Gew.-% Propanol, 4 bis 5 Gew.-% Butanol und 2 bis 4 Gew.-% Alkohole mit mindestens 5 Kohlenstoffatomen.

9. Verfahren nach einem der Ansprüche 1 bis 8, bei dem man die Destillation der zweiten Fraktioniierungszone in der Weise einstellt, daß 85 bis 99 % des zugeführten Kohlenwasserstoffs überdestillieren und 1 bis 15 % des zugeführten Kohlenwasserstoffs in dem Flüssigkeitsstrom am Boden zurückbleiben, der die im wesentlichen dehydratisierten Alkohole enthält, den Flüssigkeitsstrom einer ergänzenden Destillation unter einem weniger hohen Druck als in der zweiten Fraktionierungszone unterwirft, am Kopf im wesentlichen den restlichen Kohlenwasserstoff und am Boden die im wesentlichen von dem Kohlenwasserstoff befreiten wasserfreien Alkohole sammelt.

10. Verwendung der in der Stufe (c) gemäß einem der Ansprüche 1 bis 9 erhaltenen Mischung als Treibstoff oder Bestandteil eines Treibstoffs für Motoren.

## Claims

1. Process for the dehydration of an aqueous mixture of alcohols containing at least methanol and ethanol, characterized in that it comprises the following stages:

a) the aqueous mixture of alcohols is fractionated in a first fractionation zone by distillation under a pressure $P_1$ at least equal to atmospheric pressure, at the head are collected the vapours of at least 95% of the methanol and 0 to 65% of the ethanol of said aqueous mixture of alcohols and said vapours are condensed by transmitting at least part of the condensation heat to the second fractionation zone, as indicated hereinafter and at the bottom is drawn off a liquid stream of alcohols and water depleted (or free) of methanol;

b) at least one hydrocarbon having 2 to 6 carbon atoms is added to said liquid stream of alcohols and water and the resulting, hydrocarbon-containing mixture is fractionated by distillation in a second fractionation zone under a pressure $P_2$ such that the said condensation heat permits the distillation of at least most of the hydrocarbon in the second fractionation zone and at the head is collected a distillate essentially incorporating at least most of the said hydrocarbon and the water and at the bottom a liquid stream containing the substantially dehydrated alcohols and the water is separated from the hydrocarbon; and

c) at least part of the condensate of the first fractionation zone is mixed with at least part of the liquid stream drawn off at the bottom of the second fractionation zone.

2. Process according to claim 1, wherein, in stage (b), before supplying the hydrocarbon mixture and the stream of alcohols and water to the second fractionation zone, decanting takes place of an aqueous phase formed during hydrocarbon addition, said aqueous phase is eliminated and the mixture free from said aqueous phase is fed to the second fractionation zone.

3. Process according to claim 2, wherein the hydrocarbon addition takes place by countercurrent contacting of said hydrocarbon and the liquid stream of alcohols and water.

4. Process according to any one of the claims 1 to 3, wherein the first fractionation zone is regulated so as to collect at the head at least 98% of the methanol and 10 to 55% of the ethanol.

5. Process according to any one of the claims 1 to 4, wherein the hydrocarbon is essentially propane and in which the pressure is 1 to 5 bars in the first fractionation zone and 5 to 30 bars in the second fractionation zone.

6. Process according to any one of the claims 1 to 4, wherein the hydrocarbon is essentially butane and in which the pressure is 1 to 5 bars in the first fractionation zone and 3 to 20 bars in the second fractionation zone.

7. Process according to any one of the claims 1 to 6, wherein the aqueous mixture of alcohols contains 1 to 40% by weight water.

8. Process according to any one of the claims 1 to 6, wherein the aqueous mixture of alcohols contains 1 to 40% by weight water and 99 to 60% by weight alcohols, the alcohols comprising by weight and for 100 parts, 50 to 70% methanol, 15 to 20% ethanol, 7 to 10% propanol, 4 to 5% butanol and 2 to 4% alcohols having at least 5 carbon atoms.

9. Process according to any one of the claims 1 to 8, wherein the distillation of the second fractionation column is regulated so as to distil 85 to 99% of the charged hydrocarbon and leave 1 to 15% of the charged hydrocarbon in the liquid bottom stream, containing substantially dehydrated alcohols, said liquid stream undergoes complementary distillation under a lower pressure than that of the second fractionation zone, at the head essentially residual hydrocarbon is collected and at the bottom the essentially hydrocarbon-free anhydrous alcohols.

10. Use of the mixture obtained in stage (c) of any one of the claims 1 to 9 as fuel or a fuel constituent for engines.

FIG.1

FIG.2